# EUROPEAN PATENT SPECIFICATION

(11) **EP 1 893 033 B1**
(45) Date of publication and mention of the grant of the patent: **22.10.2008**
(21) Application number: 07787191.1
(22) Date of filing: 06.07.2007
(51) Int. Cl.: A23C 11/10, A23L 1/105, C12N 1/04

(54) **METHOD FOR PREPARING A DAIRY ANALOGUE**
VERFAHREN ZUR HERSTELLUNG EINES MILCHPRODUKT-ANALOGS
PROCÉDÉ DE PRÉPARATION D'UN ANALOGUE DE PRODUIT LAITIER

(30) Priority: 07.07.2006 EP 06447086
(43) Date of publication of application: 05.03.2008
(73) Proprietor: Alpro NV, 8560 Wevelgem (BE)
(72) Inventor: DE SCHINKEL, Dominiek, 9100 Sint-niklaas (BE); DE BUYSER, Dirk, 8210 Zedelgem (BE)
(74) Representative: Brants, Johan P.E.
(86) International application number: PCT/EP2007/056909
(87) International publication number: WO 2008/003782

(56) References cited:
- WO-A-02/37984
- WO-A-98/54296
- FR-A1- 2 831 395
- JP-A- 2005 224 224
- US-A1- 2005 069 562
- HEENAN C N ET AL: "GROWTH MEDIUM FOR CULTURING PROBIOTIC BACTERIA FOR APPLICATIONS IN VEGETARIAN FOOD PRODUCTS" LEBENSMITTEL WISSENSCHAFT UND TECHNOLOGIE, ACADEMIC PRESS, LONDON, GB, vol. 35, no. 2, 2002, pages 171-176, XP008012512 ISSN: 0023-6438
- HEENAN CN, ADAMS MC, HOSKEN RW, FLEET GH: "Survival and sensory acceptability of probiotic microorganisms in a nonfermented frozen vegetarian dessert" LEBENSM.-WISS. U. TECHNOL., vol. 37, 2004, pages 461-466, XP002412621

## Description

### Field of the invention

The present invention relates to the field of food technology. The present invention provides a bacteria culture containing lactic acid bacteria that have been adapted by growing on a 100% animal-free adaptation medium, and to a method for preparing such bacteria culture. The present invention also relates to a method for preparing dairy analogues and dairy analogues thereby obtained using said bacteria culture.

### Background

Lactic acid bacteria (LAB) are a group of gram-positive bacteria that produce lactic acid as a result of carbohydrate fermentation. They have been used for centuries in the fermentation of foods, not only for flavor and texture development, but also because of their ability to produce antimicrobial compounds, which prevent the growth of spoilage or pathogenic microorganisms.

LAB are best known for their role in the preparation of fermented food products, such as yogurt (*Streptococcus* spp. and *Lactobacillus* spp.), cheeses (*Lactococcus* spp.) and, sauerkraut (*Leuconostoc* spp.). In addition, LAB are also used for pickling of vegetables, baking, wine-making, curing fish, meats and sausages. Their growth lowers the pH due to lactic acid production. This acidification process inhibits the growth of most other microorganisms including the most common human pathogens, thus allowing these foods to obtain prolonged shelf life. The acidity also changes the texture of the foods due to precipitation of some proteins, and the biochemical conversions involved in growth enhance the flavor.

LAB are heterotrophic and generally have complex nutritional requirements because they lack many biosynthetic capabilities. Most species have multiple requirements for amino acids and vitamins. Because of this, lactic acid bacteria are generally abundant only in communities where these requirements can be provided. They are often associated with animal oral cavities and intestines (eg. *Enterococcus faecalis*), plant leaves (*Lactobacillus, Leuconostoc*) as well as decaying plant or animal matter such as rotting vegetables, fecal matter, compost, etc.

In the prior art, lactic acid bacteria are traditionally grown and maintained on media containing animal proteins such as milk, casein, meat peptone, horse serum, bovine serum albumin, cheese whey, etc., and/or animal derived ingredients such as hemin chloride, and/or ingredients obtained by using enzymes of animal origin such as trypsin, pepsin, or pancreatin. Moreover, it is well known in the art to cryo-preserve lactic acid bacteria by using animal components such as horse serum.

For example, FR 2831395 discloses the use of a deposited *Lactobacillus plantarum* strain for the fermentation of soya juice and/or any other similar soya based product. The *L. plantarum* strain is used for the fermentation of soymilk for the preparation of vegetable dairy analogues. This strain is preserved in liquid nitrogen on a medium containing animal-derived ingredients (MRS broth).

However, when using LAB that have been preserved, grown and/or maintained on media as indicated above for preparing dairy analogues, several problems may be encountered, including relatively long fermentation times, off-flavors, loss of specific metabolic activity of the LAB, less survival of the lactic acid bacteria in the prepared dairy-free products and/or limited shelf life of the prepared dairy-free products.

In addition, the use of LAB that have been preserved, grown and/or maintained on media as indicated above in vegetarian or vegan foods is an ethical problem for specific consumer groups such as vegetarians and vegans and several religious groups such as in case of beef meat peptone for Jews and Hindi or in case of pork meat peptone for Muslims.

The present invention aims to provide a solution to at least some of the above-mentioned problems.

More in particular, it is an object of the present invention to provide an improved method for preparing food products, e.g. fermented dairy analogues, which is more effective and requires shorter fermentation times.

It is further an object of the invention to provide an improved method for preparing food products, preferably fermented products, wherein said food products, e.g. dairy analogues, show improved quality, nutritional and/or organoleptic properties.

It is a further object of the present invention to provide an improved bacteria culture of lactic acid bacteria for use in the preparation of food products, preferably fermented food products such as fermented dairy analogues.

The use of growth media free of animal proteins for graving lactic acid bacteria has been reported in Heenan C N et al, Lebensm.-Wiss, u. Technol. 35: 171-176 (2002)

### Summary of the invention

In a first aspect, the invention relates to a method for preparing a dairy analogue. The method comprises the steps of:
a) isolating one or more lactic acid bacteria from their natural environment or from a culture collection on a suitable animal-free isolation medium;
b) adapting said isolated bacteria by growing said bacteria on a suitable animal-free adaptation medium;
c) culturing said adapted bacteria in a suitable animal-free culture medium, and
d) preparing a dairy analogue by adding to said dairy analogue as a starting material a suitable amount of bacteria obtained in step c).

The method is characterized in that steps a) to d) are carried out under animal-free conditions. In a preferred embodiment, the isolation, adaptation and culture media are 100% vegetable media. Preferably step d) comprises fermenting a dairy analogue by adding to said dairy analogue as a starting material a suitable amount of bacteria obtained in step c).

In another further aspect, the invention also encompasses a dairy analogue which is obtainable by the present method. The dairy analogue preferably is a vegetable food product, a vegetable food ingredient, or a vegetable functional food. More preferably, the dairy analogue is a fermented dairy analogue, which is preferably derived from soya.

The present invention also relates to lactic acid bacteria or a bacteria culture comprising one or more lactic acid bacteria that have been isolated and adapted by growing on an animal-free or on a 100% vegetable isolation and adaptation medium, respectively. The lactic acid bacteria are preferably selected from the genera of *Lactobacillus, Lactococcus*, *Bifidobacterium,* and *Streptococcus.*

The present invention provides a method for preparing a dairy analogue, preferably a fermented dairy analogue, wherein isolation, adaptation, culturing and preparation (i.e. addition and/or fermentation) steps are all performed in animal-free conditions and/or media. Therefore, in accordance with the present method contamination of compounds of animal origin is avoided during the complete production process of the dairy analogue. In addition, the applicant has shown that by growing the present bacteria on a 100% vegetable adaptation medium, the bacteria are able to undergo (bio)chemical adaptation, e.g. by changing enzyme expression patterns. Such adaptation has unexpected and advantageous consequences for the preparation process as such, but also for the qualitative and nutritional properties of the prepared dairy analogues.

With the insight to better show the characteristics of the invention, some preferred embodiments and examples are described hereafter referring to the enclosed drawings.

### Description of the figures

**FIG. 1** illustrates the pH course in a soya-milk that is being fermented with *Lactococcus lactis* S (LMG P-23669).
**FIG. 2** represents SDS PAGE profiles of *L*. *casei* T (LMG P-23506) on S medium (**FIG. 2A**), *L. casei* T (LMG P-23506) on MRS medium (**FIG. 2B**) and of *L*. *casei* 6904 on MRS medium **(****FIG. 2C****).**
**FIG. 3** shows the gradual transition in typical IR spectrum when comparing *L*. *casei* V (LMG P-23504) on S medium (VS), adapted on S medium and re-adapted on MRS medium (VSM), adapted on MRS medium and re-adapted on S medium (VMS) and adapted to MRS medium (VM).
**FIG. 4** represents a 2D-SDS PAGE profiles of *L*. *casei* V (LMG P-23504) on S medium (**FIG. 4A**), *L*. *casei* V (LMG P-23504) on MRS medium **(****FIG. 4B****).** Arrows indicate the differences between both gels.
**FIG. 5** illustrates the speed of fermentation of a soy-milk + 2% glucose when using *Lactococcus lactis* S (LMG P-23669) isolated, adapted and maintained on S or MRS medium.
**FIG. 6** illustrates the speed of fermentation of a soy-milk + 2% glucose when using *L*. *casei V* (LMG P-23504) isolated, adapted and maintained on S or MRS medium.
**FIG. 7** illustrates the speed of fermentation of a soy-milk + 2.5% raffinose when using *B*. *infantis* (LMG P-24096) isolated, adapted and maintained on S or MRS medium.
**FIG. 8** illustrates fermentation at a constant pH of 5.9 of a soy-milk + 2.5% raffinose when using *B. infantis* (LMG P-24096) isolated, adapted and maintained on S or MRS medium.

### Detailed description of the invention

The present invention provides a method for preparing a dairy analogue, preferably a fermented dairy analogue. Traditionally, preparation or fermentation processes comprise the steps of A) growing bacteria, B) culturing said bacteria and C) using said bacteria as bacteria culture in a preparation or fermentation step.

In the art, fermented and non fermented products have been sold to consumers under a so-called "100% vegetable" label. It shall however be clear that such products are in fact not 100% vegetable, since in practice, generally not all steps in the production process of such products are carried out under vegetable conditions. Traditionally, before being multiplied and inoculated as ferment, the lactic acid bacteria are grown on media containing animal proteins, e.g. as milk, casein, meat peptone, horse serum, bovine serum albumin, cheese whey, etc.

In contrast, the present invention differs from such prior art processes in that before being cultured and inoculated as bacteria culture, the lactic acid bacteria are isolated on an animal-free isolation medium and grown on an animal-free adaptation medium. In the present process isolation, adaptation, culturing and preparation or fermentation steps are all performed in animal-free conditions and/or media.

The term "*animal-free*" in this context refers to conditions and/or media wherein components of animal origin are not added. Unintended traces of components of animal origin may be present but do not have any effect on the metabolism and/or enzyme expression pattern of the lactic acid bacteria. Preferably the lactic acid bacteria are isolated and grown according to the invention in an animal-free medium which is a substantially, and preferably a 100%, vegetable medium. The term "*substantially*" as used in this context refers to a vegetable medium wherein only 100 % vegetable ingredients are used but in which unintended traces of components of animal origin may be present, but wherein such traces do not have any effect on the metabolism and/or enzyme expression pattern of the lactic acid bacteria. In a preferred embodiment, the animal-free or 100% vegetable medium comprise vegetable components and/or synthetic components derived from vegetable sources. Preferred examples of 100 % animal-free media include media wherein for instance the nitrogen source is a vegetable peptone such as soya peptone and the carbon source is a sugar moiety derived from plants such as fructose, or synthetic media with a defined amino acid composition and synthetic vitamins, which are only derived from non-animal sources.

The present invention provides a method for preparing a fermented dairy analogue which is safer and more transparent, since contamination of compounds of animal origin is avoided during the complete production process of the dairy analogue, including the isolation, adaptation, culturing and fermentation step.

The applicants have further found that by using lactic acid bacteria that have been isolated and adapted in a 100% vegetable medium, reduced fermentation times are obtained for preparing fermented dairy analogues. Moreover, survival of the lactic acid bacteria during shelf life of prepared dairy analogues is also increased.

Furthermore, using lactic acid bacteria that have been isolated and adapted in a 100% vegetable medium as a bacteria culture in the present method permits to improve structural, nutritional and/or organoleptic properties of prepared dairy products, including improved digestibility.

Another advantage of the present bacteria culture is that it does not contain compounds of animal origin. The bacteria culture can advantageously be used to prepare dairy analogues that are suitable for specific consumer groups such as vegetarians, vegans, or religious groups such as Jews or Muslims. Furthermore, use of the present bacteria cultures removes the risk of transmitting animal derived contamination, such as bovine spongiform encephalopathy (BSE) or other infectious and harmful agents, into consumers of the prepared dairy analogues.

The present method will be further explained below with reference to the different steps.

### Isolation of Lactic acid bacteria

A first step in the present method comprises the isolation of one or more lactic acid bacteria on an animal-free isolation medium.

Lactic acid bacteria can be isolated from their natural environment, e.g. from animal oral cavities and intestines, from milk, cheese; from plant leaves as well as decaying plant or animal matter such as decaying vegetables, fecal matter, compost, naturally fermented vegetable products (pickles, sauerkraut, etc.).

Lactic acid bacteria can also be isolated from a culture collection, such as culture collections that are present in depository institutions under the Budapest treaty.

Bacteria are isolated on an isolation medium, and preferably a 100% vegetable medium. The isolation step in the present method comprises the *single step* of bringing the bacteria on a suitable isolation medium, preferably a 100% vegetable isolation medium. The isolation step is the first step of bringing bacteria, e.g. from the environment or from a culture collection, onto a suitable isolation medium as defined herein. Thus, isolation in principle comprises substantially one handling step.

Preferably the lactic acid bacteria are selected from the group genera of *Lactobacillus, Lactococcus Bifidobacterium,* and *Streptococcus.* In a preferred embodiment the lactic acid bacteria comprise *Lactobacillus acidophilus*, *L*. *bulgaricus*, *L*. *casei*, *L*. *paracasei*, *L*. *delbrueckii*, *L*. *fermentum*, *L*. *plantarum*, or *L*. *reuteri*. In another embodiment the lactic acid bacteria comprise *Lactococcus garvieae*, *L*. *lactis*, *L*. *cremoris*, *L*. *lactis* subsp. *diacetylactis*, *L*. *piscium*, or *L*. *raffinolactis*. In another embodiment the lactic acid bacteria comprise *Bifidobacterium infantis, B*. *longum, B*. *breve, B*. *animalis (lactis), and B*. *adolescentis*. In yet another embodiment the lactic acid bacteria comprise *Streptococcus salivarius* or *Streptococcus thermophilus,* and preferably *Streptococcus salivarius* ssp. *thermophilus*.

In a more preferred embodiment, the lactic acid bacteria comprise *Lactobacillus casei* V (LMG P-23504), *Lactobacillus casei* W (LMG P-23505), *Lactobacillus casei* T (LMG P-23506), *Lactococcus lactis* S (LMG P-23669), *Bifidobacterium infantis* S (LMG P-24096) and/or *Streptococcus salivarius* ssp. *thermophilus* (LMG P-24095). The *Lactobacillus casei* V strain has been deposited to the BCCM/LMG Bacteria Collection, Ledeganckstraat 35 B-9000 Gent, Belgium on April 11, 2006 (receipt of the deposit by the Depositary Authority on February 17, 2006). The deposited strain has the characteristics of the allocated accession number LMG P-23504. The *Lactobacillus casei* W strain has been deposited to the BCCM/LMG Bacteria Collection, Ledeganckstraat 35 B-9000 Gent, Belgium on April 11, 2006 (receipt of the deposit by the Depositary Authority on February 17, 2006). The deposited strain has the characteristics of the allocated accession number LMG P-23505. The *Lactobacillus casei* T strain has been deposited to the BCCM/LMG Bacteria Collection, Ledeganckstraat 35 B-9000 Gent, Belgium on April 11, 2006 (receipt of the deposit by the Depositary Authority on February 17, 2006). The deposited strain has the characteristics of the allocated accession number LMG P-23506. In another embodiment said lactic acid bacteria is *Lactococcus lactis* S. This strain has been deposited to the BCCM/LMG Bacteria Collection, Ledeganckstraat 35 B-9000 Gent, Belgium on May 15, 2006. The deposited strain has the characteristics of the allocated accession number LMG P-23669. In another embodiment said lactic acid bacteria is *Bifidobacterium infantis* S. This strain has been deposited to the BCCM/LMG Bacteria Collection, Ledeganckstraat 35 B-9000 Gent, Belgium on April 12, 2007. The deposited strain has the characteristics of the allocated accession number LMG P-24096. In yet another embodiment said lactic acid bacteria is *Streptococcus salivarius* ssp. *thermophilus.* This strain has been deposited to the BCCM/LMG Bacteria Collection, Ledeganckstraat 35 B-9000 Gent, Belgium on April 12, 2007. The deposited strain has the characteristics of the allocated accession number LMG P-24095.

### Adaptation

In accordance with the present invention, a method is provided wherein lactic acid bacteria are isolated and adopted by growing in a suitable animal-free isolation and adaptation medium at their optimal growth temperature.

The term "*growing*" as used herein refers to the growth of bacteria. Bacterial growth is herein defined as an increase in bacterial biomass.

The adaptation step in the present method preferably comprises growing and frequently re-plating the bacteria on a suitable adaptation medium, preferably a 100% vegetable adaptation medium. The adaptation step implies at least one and preferably more than one re-plating of the bacteria onto a suitable adaptation medium as defined herein. During this adaptation step, the bacteria are re-plated frequently, e.g. every 3 to 5 days, in order to keep the bacteria in a viable state. Preferably, during these re-plating operations, the largest bacterial colonies are selected. The bacteria are grown at a suitable temperature for a suitable time. For instance, for *Lactococcus* bacteria, the temperature is preferably around 30°C; for *Lactobacillus* the temperature is preferably 30°C or 37°C dependent on the strain, for *Streptococcus thermophilus* the temperature is preferably 42°C, while for *Bifidobacerium* the temperature is preferably around 37°.

Adaptation step is in particular characterized in that the LAB are grown on an animal-free, or 100% vegetable medium. The applicant has shown that by growing and frequently re-plating LAB on a 100% vegetable medium, LAB adapt to such medium. The term "*adaptation*", when used in the context refers to the phenomena that the LAB have undergone chemical and metabolic modifications, especially with regard to their enzyme metabolism, e.g. by adjusting their enzyme pattern, induction/down regulation of certain enzymes, enzyme activity, enzyme synthesis kinetics, etc..., in function of the components present in the vegetable medium. As a result thereof, the LAB are more adapted to vegetable components, and obtain improved capabilities to subsequently ferment products of vegetable origin.

Any standard medium for the culture of bacteria may be used as the basis for preparing the present vegetable medium, insofar as the final composition of the medium is animal-free, or is substantially vegetable, as defined herein. The term "media" may be used in reference to solid plated media which support the growth of the bacteria. Also included within this definition are semi-solid and liquid microbial growth systems, as well as any type of media. Standard solid media can be prepared from any liquid media by the addition of a solidifying agent such as agar.

The medium of the present invention preferably comprises a carbon source, a nitrogen source, a vitamin source, an essential mineral source and optionally a selecting agent, such as a reducing agent as L-cysteïn or an antibiotic, for selecting the microorganisms to be cultured.

In one embodiment the animal-free isolation medium and the animal-free adaptation medium are a same medium. In another embodiment, the animal-free isolation medium and the animal-free adaptation medium are different media. For example, the adaptation medium can be poorer than the isolation medium, or may comprise components (such as for instance specific peptides derived from a vegetable source, raffinose, stachyose, phytic acid, ...) which improve the adaptation process. In an example, the isolation medium comprises different fermentable carbohydrates such as fructose, glucose and sucrose and a nitrogen source originating from various vegetable peptones (e.g. from soya and potato), while the adaptation medium is more selective and comprises for instance a specific soya peptone with a specific molecular weight distribution and one fermentable carbohydrate (e.g. fructose).

As used herein, the term "*carbon source*" is used in reference to any compound which may be utilized as a source of carbon for bacterial growth and/or metabolism. Carbon sources may be in various forms, including, but not limited to polymers, carbohydrates, acids, alcohols, aldehydes, ketones, amino acids, and peptides.

As used herein, the term "*nitrogen source*" is used in reference to any compound which may be utilized as a source of nitrogen for bacterial growth and/or metabolism. As with carbon sources, nitrogen sources may be in various forms, such as free nitrogen, as well as compounds which contain nitrogen, including but not limited to amino acids, peptones, vitamins, ammonia and nitrogenous salts.

As used herein, the term "*mineral source*" may reference to the addition of one or more minerals. As used herein, the term "*vitamin source*" may reference to the addition of one or more vitamins.

As used herein, the term "*selecting agent*" is used in reference to any compound which inhibits the growth of, or kills microorganisms or stimulates the growth of the selected organism. It is intended that the term be used in its broadest sense, and includes, but is not limited to compounds such as L-cystein or antibiotics which are produced naturally or synthetically. It is also intended that the term includes compounds and elements that are useful for inhibiting the growth of, or for killing microorganisms or for stimulating selected organisms. Selecting agents may be incorporated into media used in the present invention. In this manner, organisms with undesirable characteristics (e.g., contaminants) may be inhibited or killed prior to or during preservation and/or revival of the organisms of interest. Alternatively, by adding stimulating agents, such agents stimulate growth of selected organisms so that the selected organisms have an advantage during their revival.

In a preferred embodiment, the medium according to the invention comprises a vegetable peptone; a yeast component (such as a yeast extract); a vegetable derived fermentable carbohydrate; and a buffer.

Vegetable peptones are complex mixtures of organic and inorganic compounds that are obtained by digestion of protein-containing tissues of plants such as e.g. soybean meal or soybean protein. Peptones primarily contain peptides and single amino acids. Being crude digests of complex materials, they contain a great variety of other organic and inorganic materials. The peptones may be selected from the group comprising soya, cotton, wheat, malt, corn, potato, bean, lupin, sorghum and/or rice. Preferably the peptone is a soya peptone. Suitable examples of vegetable peptones include but are not limited to a commercial papaic digest of soybean protein (soybean meal), a commercial papaic digest of wheat flour, a commercial papaic digest of potato protein, etc...

Standard procedures well known to those skilled in the art can be used for the preparation of vegetable peptones. For instance, soy bean derived protein compositions can be prepared by enzymatic digestion of soy bean meal or soy isolate using standard vegetable enzymes such as papain. Soy bean derived protein compositions can also be obtained by acid hydrolysis of a soy isolate. To sum up, vegetable peptones are chemical or enzymatic digests of vegetable proteins and contain a mixture of amino acids, small peptides and polypeptides of different size. Preferably said vegetable peptone is present in the medium in an amount of between 0.1 and 10 % by weight, and preferably between 0.5 and 5 % by weight, and more preferably of about 1 % by weight.

In another embodiment, the medium comprises a yeast component. Such yeast component may be used as a source of amino acids, vitamins, coenzymes and purines and pyrimidines including many needed as growth factors by organisms. The concentration of yeast component in the present medium is preferably comprised between 0.1 and 10 % by weight, and preferably between 0.3 and 5 % by weight, and more preferably of about 0.5% by weight. Yeast components can be prepared by standard procedures well known to those skilled in the art. Furthermore, yeast components are commercially available. The yeast component may consist of an autolyzed yeast, yeast extract (i.e. an extract of yeast cells) or an (ultra)filtrated yeast extract.

In a further embodiment, the present vegetable isolation and/or adaptation medium comprises a suitable buffer to maintain the optimum pH range of the organism in a solid medium or liquid medium in order to prevent marked changes in pH which otherwise would result from microbial production of organic acids or bases. Examples of suitable buffers include but are not limited to sodium and potassium phosphates and calcium carbonate. Crude organic preparations such as peptones (see below) also act as buffers. Chemical components such as tricine and MOPS may also be used as buffer components. The concentration of buffer in the present medium is preferably comprised between 0.1 and 10 % by weight; and preferably between 0.3 and 5 % by weight, and preferably about 0.5% by weight. Preferably the vegetable medium is buffered to a pH of between 5 and 8, and preferably between 6 and 6.5.

In an example the vegetable medium is buffered to a pH of 6.3 in the case of a medium for *Lactococcus lactis* or *Lactobacillus casei.*

In yet a preferred embodiment, the present invention provides an isolation and/or adaptation medium which comprises a vegetable derived fermentable carbohydrate. Carbohydrates are chemical compounds that contain oxygen, hydrogen and carbon atoms. The term "vegetable derived" in this context refers to carbohydrates, which is a large group of sugars, starches, celluloses, and gums, that are derived from a vegetable source such as corn, wheat, chicory, tapioca, rice, potato, beet or cane and that are substantially animal free. The fermentable carbohydrates may include glucose, fructose, sucrose, galactose, etc... This term excludes carbohydrates that are derived from milk or milk products, such as e.g. lactose. Preferably fructose or glucose is used as carbohydrate. The concentration of fermentable carbohydrate in the present medium is preferably comprised between 0.1 and 10 % by weight, and preferably between 0.5 and 5 % by weight, and preferably of 1 % by weight.

For preparing a solid medium, the vegetable medium further contains agar-agar, preferably in an amount of between 1 to 2% by weight, and for instance in an amount of 1.5% by weight.

### Culturing

Step c) of the present method comprises the culturing of adapted bacteria in a suitable animal-free culture medium.

Such animal-free culture medium may comprise vegetable peptones as defined herein, e.g. from soya, wheat, rice, potato; yeast components as defined herein; vegetable derived fermentable carbohydrates as defined herein; or complex carbohydrate sources such as corn steep liquor, molasses, etc...

Culturing involves the multiplication of adapted bacteria on a larger scale with the aim to obtain sufficient biomass which can be used in a next step for preparing dairy analogues.

It will be clear that temperature and time required for the culturing step will depend on the type of lactic acid bacteria. Also, the culturing is carried out at the optimal temperature of the respective adapted bacteria, e.g. for *Lactococcus lactis* S at 30°C whereas for *Lacfobacillus casei* V at 30° or 37°C, depending on the strain. pH is followed and kept constant by addition of alkaline like sodium hydroxide, ammonium hydroxide, calcium hydroxide, ... Optimal pH depends on the culture and is preferably comprised between 5 and 8. The pH is for instance about 6.3 in the case of *Lactococcus lactis* S or *Lactobacillus casei* V.

### Preparation of dairy analogues

In a following step d), a dairy analogue is prepared by adding to said dairy analogue as a starting material a suitable amount of bacteria that has been cultured (multiplied) in step c). A suitable amount may comprise between 10⁵ and 10¹⁰ bacteria per ml, and preferably comprise 10⁷ bacteria/ml. The preparation step d) may or may not include a fermentation step: the bacteria from step c) can be added as an ingredient to the dairy analogue without fermentation or can be added as a starter culture which is subsequently fermented. Optionally, the product to be fermented may be pasteurized or sterilized prior to the fermentation thereof.

In a preferred embodiment, the present invention provides a method for preparing a dairy analogue, wherein fermentation time during the preparation of said dairy analogue is reduced. In a preferred embodiment, a method is provided wherein fermentation time during the preparation of said dairy analogue is reduced with at least 10%, preferably with at least 15%, more preferably with at least 20% and even more preferably with at least 30%. The fermentation time may for instance be reduced with 15, 18, 20, 22, 25, 27, 30, 35, 40, 45 or even 50%.

It will be clear that temperature and time required for the fermentation step will depend on the type of dairy analogue to be fermented. In a preferred example the dairy analogue is soya milk, prepared from soybeans as described as Tonyu or prepared from Soy Isolates. Also, the fermentation is carried out at the optimal temperature of the respective adapted bacteria, e.g. for *Lactococcus lactis* S at 30°C whereas for *Lactobacillus casei* V at 30° or 37°C, depending on the strain. During fermentation, pH decreases and proteins coagulate. Example 3 illustrates how the use of a culture of lactic acid bacteria that have been isolated, adapted and cultured according to the invention permits to improve the preparation of a dairy analogue, and in particular to accelerate its fermentation and thus to reduce fermentation time during the preparation of such dairy analogue.

In a preferred embodiment, the present invention may comprise the further step(s) of mixing of the fermented product in order to obtain a soft and smooth product, and/or admixing one or more additional ingredients such as for example pieces of fruit(s) or mashed fruit(s), vegetables, herbs, spices, sugar and sweeteners, flavors, colorants, stabilizers and thickeners, salt, preservatives,...

### Dairy analogues

Lactic acid bacteria that have been isolated and adapted according to steps a) and b) of the present method and/or a bacteria culture comprising such lactic acid bacteria can advantageously be used in food preparation processes, and for instance in food fermentation processes, and preferably for preparing animal-free food preparations such as a fermented dairy analogue.

The term "*dairy analogue*" as used herein is meant to refer to a product of vegetable origin and may include a vegetable food product, a vegetable food ingredient, or a vegetable functional food. The term "*of vegetable origin*" indicates that the product only contains compounds derived from plants.

The term "*food*" or "*food product*" is used herein in a broad sense, and covers food for humans as well as food for animals (i.e. a feed). In a preferred aspect, the food is for human consumption. The food may be in the form of a solution or as a solid, depending on the use and/or the mode of application and/or the mode of administration.

Non limitative examples of "*vegetable food products*" which may be obtained using a bacteria culture according to the present invention include for instance, yoghurts and drinking yoghurts; cheese, cheese sauce, (sour) cream, whipping cream, whipped topping, ice cream, water ices and desserts, confectionery, biscuits cakes and cake mixes, snack foods, fruit fillings, cake glaze, chocolate bakery filling, cake fillings, cake and doughnut icing, instant bakery filling creams, filing for cookies, ready-to-use bakery filling, reduced calorie filling, nutritional beverage, acidified soy/juice beverage, beverage powders, calcium fortified soymilk, aerated frozen desserts. In a preferred embodiment, preferably the present invention may be used in connection with soya based yoghurt and cheese production, such as fermented soya yoghurt drink, soya yoghurt, soya drinking yoghurt, soy cheese, fermented soy cream, soya based desserts and others.

The term "*vegetable food ingredient*" as used herein includes a formulation which is or can be added in the preparation of other foodstuffs. The food ingredient may be in the form of a solution or as a solid-depending on the use and/or the mode of application and/or the mode of administration. As an example a fermented soy cream can be used as ingredient for the preparation of ready-meals; a fermented soy yoghurt can be used as an ingredient for soya milk shake or for soya ice cream which is based on soya yoghurt.

As used herein, the term "*vegetable functional food*" means vegetable food which is capable of providing not only a nutritional effect and/or a taste satisfaction, but is also capable of delivering a further beneficial effect to a consumer. Accordingly, functional foods are ordinary foods that have components or ingredients (such as those described herein) incorporated into

them that impart to the food a specific functional, e.g. medical or physiological benefit, other than a purely nutritional effect. Some functional foods are nutraceuticals. Here, the term "nutraceutical" means a food which is capable of providing not only a nutritional effect and/or a taste satisfaction, but is also capable of delivering a therapeutic (or other beneficial) effect to the consumer. Nutraceuticals cross the traditional dividing lines between foods and medicine. As an example an animal-free *Bifidobacterium* preparation can be cited, which is added to soy yoghurt or fruit preparations for the preparation of a vegetable functional food.

Such a vegetable functional food could be a nutraceutical in that it contributes to a better gut health, lowering cholesterol, stimulate immunity, etc.

The invention relates to a dairy analogue obtainable by the present method. The present dairy analogue can be characterized as follows.

In one embodiment a dairy analogue is provided having a reduced amount of phytic acid. A dairy analogue is provided wherein more phytic acid has been degraded during fermentation than in a dairy analogue not obtained by a method according to the invention. The invention thus provides a dairy analogue wherein degradation of phytic acid is higher, and preferably at least 10%, more preferably at least 25% and even more preferably at least 50% higher, than in a dairy analogue not obtained by a method according to the invention. In a preferred embodiment, a dairy analogue is provided wherein at least 10%, preferably at least 15%, more preferably at least 20% and even more preferably at least 25% more phytic acid is degraded than in a dairy analogue not obtained by a method according to the invention. In an example a dairy analogue is provided wherein degradation of phytic acid is at least 1.5 times, and preferably at least 2 times, or even at least 3 times higher than the degradation of phytic acid in a dairy analogue not obtained by a method according to the invention.

In another embodiment a dairy analogue is provided having a reduced amount of raffinose. A dairy analogue is provided wherein more raffinose has been degraded during fermentation than in a dairy analogue not obtained by a method according to the invention. The invention thus provides a dairy analogue wherein degradation of raffinose is higher, and preferably at least 10%, more preferably at least 25% and even more preferably at least 50% higher, than in a dairy analogue not obtained by a method according to the invention. In a preferred embodiment, a dairy analogue is provided wherein at least 25%, preferably at least 50%, more preferably at least 75% and even more preferably at least 100% more raffinose is degraded than in a dairy analogue not obtained by a method according to the invention. In an example a dairy analogue is provided wherein degradation of raffinose is at least 3 times, and preferably at least 5 times, or even at least 7 times higher than the degradation of raffinose in a dairy analogue not obtained by a method according to the invention.

In yet another embodiment, a dairy analogue is provided having an increased amount of aglucon forms of isoflavones. A dairy analogue is provided wherein more glucoside form(s) of isoflavones have been transformed during fermentation into aglucon form(s) of isoflavones than in a dairy analogue not obtained by a method according to the invention. The invention thus provides a dairy analogue wherein transformation of glucoside form(s) of isoflavones into aglucon form(s) of isoflavones is higher, and preferably at least 10%, more preferably at least 25% and even more preferably at least 50% higher, than in a dairy analogue not obtained by a method according to the invention. In a preferred embodiment, a dairy analogue is provided wherein at least 5%, preferably at least 10%, more preferably at least 20% and even more preferably at least 25% more transformation of glucoside form(s) of isoflavones into aglucon form(s) of isoflavones than in a dairy analogue not obtained by a method according to the invention. In an example, a dairy analogue is provided wherein transformation of a glucoside form into an aglucon form of isoflavones is at least 1.1 times, and preferably at least 1.2 times, or even at least 1.3 times higher than in a dairy analogue not obtained by a method according to the invention.

### Lactic acid bacteria

In another embodiment the invention relates to lactic acid bacteria that have been isolated and adapted by growing on a 100% vegetable isolation and adaptation medium, respectively.

In a preferred embodiment, the invention provides lactic acid bacteria as disclosed herein showing an increase in activity of one or more enzymes compared to lactic acid bacteria that have not been grown on a 100% vegetable isolation and adaptation medium. Preferably said enzyme(s) is (are) selected from the group comprising acid phosphatase, alkaline phosphatase, α-chymotrypsin, α-galactosidase and β-glucosidase.

In a preferred embodiment, the invention provides lactic acid bacteria as disclosed herein showing an increase in activity with at least 5%, preferably at least 10%, preferably with at least 15%, preferably with at least 20% and more preferably with at least 30% of one or more of the above given enzymes. Enzyme activity may for instance be increased with 15, 18, 20, 22, 25, 27, 30, 35, 40, 45 or even 50%.

In one preferred embodiment, the invention provides lactic acid bacteria as disclosed herein, which are capable of increasing degradation of phytic acid in a dairy analogue during fermentation thereof. The invention thus provides lactic acid bacteria showing an increase in phosphatase activity, e.g. of acid phosphatase and/or alkaline phosphatase activity, with at least 5%, preferably with at least 10%, preferably with at least 20% and more preferably with at least 30%. For instance, lactic acid bacteria are provided that are capable of degrading at least 1.5 times, and preferably at least 2 times, or even at least 3 times more phytic acid than lactic acid bacteria that have not been grown on a 100% vegetable isolation and adaptation medium in accordance with the present invention.

In another preferred embodiment, the invention provides lactic acid bacteria as disclosed herein, which are capable of increasing degradation of raffinose in a dairy analogue during fermentation thereof. The invention thus provides lactic acid bacteria showing an increase in α-galactosidase activity with at least 5%, preferably with at least 10%, preferably with at least 20% and more preferably with at least 30%. For instance lactic acid bacteria are provided that are capable of degrading at least 3 times, and preferably at least 5 times, or even at least 7 times more raffinose than lactic acid bacteria that have not been grown on a 100% vegetable isolation and adaptation medium in accordance with the present invention.

In yet another embodiment, the invention provides lactic acid bacteria as disclosed herein, which are capable of increasing transformation of (inactive) glucoside form(s) into (active) aglucon form(s) of isoflavones in a dairy analogue during fermentation thereof. The invention thus provides lactic acid bacteria showing an increase in β-glucosidase activity, with at least 5%, preferably with at least 10%, preferably with at least 20% and more preferably with at least 30%. For instance lactic acid bacteria are provided that are capable of transforming at least 1.1 times, and preferably at least 1.2 times, or even at least 1.3 times more glucoside form(s) into aglucon form(s) of isoflavones than lactic acid bacteria that have not been grown on a 100% vegetable isolation and adaptation medium in accordance with the present invention.

In another embodiment, the invention further relates to a bacteria culture comprising one or more lactic acid bacteria that have been isolated and adapted by growing on a 100% vegetable isolation and adaptation medium, respectively, and preferably an isolation and adaptation medium as defined herein. The invention further preferably relates to a bacteria culture comprising one or more lactic acid bacteria as defined above.

A bacteria culture or lactic acid bacteria as defined herein are particularly suitable for being used for improving the preparation of dairy analogue. The preparation can be improved in the sense that dairy analogues can be obtained having improved quality, nutritional and organoleptic properties, such as for instance, enhanced taste and/or flavor stability, enhanced digestibility, improved nutritional value, enhanced availability of minerals such as Ca, Fe, Mg, etc... The invention for instance relates to the use of a bacteria culture or lactic acid bacteria as defined herein for preparing a dairy analogue having a reduced amount of phytic acid and/or raffinose, and/or for preparing a dairy analogue having an increased amount of aglucon form(s) of isoflavones.

In addition the preparation process of the dairy analogue as such can also be improved. In particular, the invention also relates to the use of a bacteria culture or lactic acid bacteria as defined herein for reducing fermentation time during the preparation of a dairy analogue. Preferably a bacteria culture or lactic acid bacteria according to the invention are used for reducing fermentation time during the preparation of a dairy analogue with at least 10%, preferably with at least 15%, preferably with at least 20% and more preferably with at least 30%. The fermentation time may for instance be reduced with 15, 18, 20, 22, 25, 27, 30, 35, 40, 45 or even 50%.

The invention further relates to the use of lactic acid bacteria or a culture thereof as disclosed herein for increasing degradation of phytic acid in a dairy analogue during fermentation thereof, and/or for increasing degradation of raffinose in a dairy analogue during fermentation thereof and/or for increasing transformation of glucoside form(s) into aglucon form(s) of isoflavones in a dairy analogue during fermentation thereof.

The following examples are intended to illustrate particular embodiments of the invention, and do not limit the scope of the invention.

### Examples

### Example 1: 100% vegetable adaptation medium according to the present invention

The present example illustrates a 100% vegetable medium according to the present invention. The medium consists of: 10 g Peptone S (= commercial papaic digest of soybean meal from Acumedia, 5 g Yeast Extract, ultrafiltrated ( Ultrafiltrated autolysate of bakers' yeast) from Acumedia, 10g Fructose, 5 g of a Phosphate buffer pH=7,2 (from Acumedia) 15 g Agar-agar ( bacteriological), and demineralised water up to 1 liter.

For preparing broth, the above-mentioned medium wherein agar-agar is omitted may be used.

### Example 2: Characterization of bacteria cultures according to the present invention

Lactic acid bacteria that were used in the following examples as starting material were either isolated from natural sources (e.g. food products) or obtained from a culture collection (BCCM - Gent Belgium).

### 2.1 Enzyme induction pattern

### * Lactobacillus casei V (LMG P-23504)

In a first experiment *Lactobacillus casei* V (LMG P-23504) was grown and maintained in the following ways: 1) grown and maintained on a S medium (VS); 2) grown and maintained on a MRS medium (VM); 3) grown and maintained on a S medium and once re-plated on a MRS medium (VSM); and 4) and grown and maintained on a MRS medium and once re-plated on a S medium (VMS).

The S medium is a substantially vegetable medium according to the invention comprising soya peptone, yeast extract, a phosphate buffer and a sugar source. The MRS medium corresponds to medium comprising a meat peptone. The LAB were re-plated twice a week on the MRS or the S media whereby a selection was made of the largest bacterial colonies (=breeding).

The enzyme induction patterns of the bacteria grown on the above-stated media were analyzed in a semi-quantitative way. Using a commercial kit (Biomérieux) enzyme activity was determined by means of a color code (0: no enzyme activity to 5: strongest enzyme activity). Enzyme activities were measured after incubation at 37° during 4 hours.

Results of this experiment indicated that by adapting *Lactobacillus casei* V (LMG P-23504) to a substantially vegetable medium (S medium) according to the invention, certain enzymes were significantly induced or increased, e.g. alkaline phosphatase, acid phosphatase, β-glucosidase and α-fucosidase. These enzymes were practically not induced when adapting the bacteria to a MRS medium. However, when the bacteria, adapted to a substantially vegetable medium were re-adapted to a MRS medium, there was a transition in the enzyme pattern: the enzyme induction pattern was reversed.

The following example illustrates the increased phosphatase activity of the *L*. *casei* V (LMG P-23504) when grown and maintained on S medium, compared to *L*. *casei* V (LMG P-23504) grown and maintained on MRS medium. Soy milk + 2% added glucose was inoculated with 1X10⁷/ mL of *L*. *casei* V that was maintained on S or that was maintained on MRS medium and the inoculated soy milk was fermented at 30° until pH of 4.5.

The content of phytic acid was measured in the soy milk before and after fermentation. Results are presented in **Table 1.**

**Table 1**

| **sample** | **wt % phytic acid** | **% breakdown** |
|---|---|---|
| before fermentation | 0,15 | |
| fermented with *L*. *casei* V (MRS) | 0,09 | **40** |
| Fermented with *L*. *casei* V (S) | < 0,05 | **> 67** |

**Table 1** demonstrates that the *L*. *casei* V (LMG P-23504) that was maintained on S medium induces a higher breakdown of the anti-nutritional factor phytic acid. This is a nutritional advantage for the consumer. The amount of phytic acid in the dairy analogue was reduced with more than 67% when using *L*. *casei* V that had been maintained on S medium compared with a reduction of 40 % when using *L*. *casei* V that had been maintained on MRS medium. This example further indicates that there is more than 27% more phytic acid degraded in the dairy analogue prepared with *L*. *casei* V that was maintained on S medium than in a dairy analogue prepared with *L*. *casei* V that was maintained on MRS medium. *L. casei* V that was maintained on S medium is capable of increasing the degradation of phytic acid in a dairy analogue with a factor of at least 1.675.

### * Lactococcus lactis LMG 8522

In another experiment a *Lactococcus lactis* LMG 8522 was adapted for several months either on a S medium or on a MRS medium. After several months of culture, enzyme activity was determined using the kit as defined above.

Results hereof indicated that by adapting *Lactococcus lactis* on a substantially vegetable medium according to the invention, certain enzymes were induced, e.g. alkaline phosphatase and α-chymotrypsine. These enzymes were not induced when adapting the lactic acid bacterium on a MRS medium.

### * Lactococcus lactis S (LMG P-23669)

In yet another experiment a *Lactococcus lactis* S (LMG P-23669) was adapted for several months either on S medium or on MRS medium. After several months of culture, enzyme activity was determined using the kit as defined above.

Results hereof indicated that by adapting *Lactococcus lactis* S (LMG P-23669) on a substantially vegetable medium according to the invention, certain enzymes were induced or increased, e.g. alkaline phosphatase and α-chymotrypsine. These enzymes were not induced when adapting the lactic acid bacterium on a MRS medium.

The following example illustrates the increased phosphatase activity of *Lactococcus lactis* S (LMG P-23669) when maintained on S medium, compared to *Lactococcus lactis* S (LMG P-23669) maintained on a MRS medium. Soy milk + 2% added glucose was inoculated with 1X10⁷/ mL of *Lactococcus lactis* S that was maintained on S or that was maintained on MRS medium and the inoculated soy milk was fermented at 30° until pH of 4.5.

The content of phytic acid was measured in the soy milk before and after fermentation. Results are presented in **Table 2.**

**Table 2**

| sample | wt % phytic acid | % breakdown |
|---|---|---|
| before fermentation | 0,15 | |
| fermented with *L. lactis* S (MRS) | 0,14 | 7 |
| fermented with *L. lactis* S (S) | 0,11 | 27 |

**Table 2** demonstrates that the *L*. *lactis* S (LMG P-23669) that was maintained on S medium induces a higher breakdown of the anti-nutritional factor phytic acid. The amount of phytic acid in the dairy analogue was reduced with 27% when using *L. lactis* S that had been maintained on S medium compared with a reduction of 7% when using *L. lactis* S that had been maintained on MRS medium. This example further indicates that there is about 20% more phytic acid degraded in the dairy analogue prepared with *L*. *lactis* S that was maintained on S medium than in a dairy analogue prepared with *L. lactis* S that was maintained on MRS medium. *L. lactis* S that was maintained on S medium is capable of increasing the degradation of phytic acid in a dairy analogue with a factor of at least 3.86.

### * Bifidobacterium infantis (LMG P-24096)

In yet another experiment *Bifidobacterium infantis* LMG P-24096 was adapted for several months either on a S medium or on a MRS medium in anaerobic conditions. After several months of culture, enzyme activity was determined using the kit as defined above.

Results hereof indicated that by adapting *Bifidobacterium infantis* (LMG P-24096) on a substantially vegetable medium according to the invention, certain enzymes were induced or increased, e.g. α-galactosidase and β-glucosidase. These enzymes were not induced when adapting the lactic acid bacterium on MRS medium.

The following example illustrates for instance the increased α-galactosidase and β-glucosidase activity of the *Bifidobacterium infantis* (LMG P-24096) when maintained on S medium, compared to *Bifidobacterium infantis* (LMG P-24096) maintained on a MRS medium.

Soy milk + 2.5 % raffinose was inoculated with 1X10⁷/ mL of *B. infantis* that was maintained on S or that was maintained on MRS medium and the inoculated soy milk was fermented at 37° under anaerobic conditions.

During fermentation the content of raffinose was measured before and after 6 hours of fermentation by *B. infantis* that was maintained on S or that was maintained on MRS medium. The *B. infantis* culture adapted to the S medium showed a higher breakdown of raffinose due to the increased α-galactosidase activity: 6 hours after the start of the fermentation, *B. infantis* that was maintained on S medium had broken down 6 % of the raffinose compared with the *B. infantis* that was maintained on MRS medium and that had broken down 0.8 % of the raffinose. This example indicates that there is about 5.2% more raffinose degraded in the dairy analogue prepared with *B. infantis* that was maintained on S medium than in a dairy analogue prepared with *B. infantis* that was maintained on MRS medium. This example further indicates that *B. infantis* that was maintained on S medium is capable of increasing the degradation of raffinose in a dairy analogue with a factor of at least 7.5.

During fermentation the content of isoflavones was also measured before and after 6 hours of fermentation by *B. infantis* that was maintained on S or that was maintained on MRS medium. The *B. infantis* culture adapted to the S medium showed higher transformation of the biological inactive glucoside-form of the isoflavones to the biologically active aglucon-form due to increased β-glucosidase activity: 6 hours after the start of the fermentation, *B. infantis* that was maintained on S medium had transformed 98 % of the glycosides daidzin and genistin to the aglucons daidzein and geistein while *B. infantis* that was maintained on MRS medium had transformed only 89%. This example indicates that there is about 9% more glucoside-form of the isoflavones that are transformed in the dairy analogue prepared with *B*. *infantis* that was maintained on S medium than in a dairy analogue prepared with *B. infantis* that was maintained on MRS medium. This example further indicates that *B. infantis* that was maintained on S medium is capable of increasing transformation of (inactive) glucoside form(s) into (active) aglucon form(s) of isoflavones with a factor of at least 1.1.

Activities of the induced enzymes can be correlated to improvements with regard to quality and organoleptic properties of dairy analogues produced with the LAB.

For instance, the enzymes alkaline phosphatase and acid phosphatase may play a role in the degradation of phytic acid, which is an anti-nutritional factor present in e.g. soya. Phytic acid binds minerals such as Ca, Fe and Mg and thus reduces their bio-availability. Degradation of phytic acid therefore increases availability of such minerals.

Another example is that of β-glucosidase. This enzyme transforms glucosides to a glucose-unit and an aglucon. In a soya-matrix the β-glucosidase will transform biological inactive glucoside-form of isoflavones to biological active aglucon-moiety. An increased β-glucosidase activity increases the bio-availability of isoflavones that are present in soya in the glucoside-form.

Yet another example is that of α-galactosidase. This enzyme is capable of breaking down raffinose and stacchyose. These are undigestable sugars that are typically present in soy milk and that cannot be digested by humans. This can cause unwanted gas formation in the gastro-intestinal tract. An increased breakdown of these flatulence-sugars in soy milk increases the digestability for the consumers.

Still another example is α-chymotrypsin. This proteolytic enzyme has a function in predigestion, formation of bio-active peptides, aroma formation, structure formation.

### * Conclusion

Summarized, these results indicate that lactic acid bacteria that are adapted to a substantially vegetable medium undergo significant metabolic changes. These metabolic changes of the strain are however reversed when the strain is re-adapted to a medium containing compounds of animal origin. The induced enzymes play a role in the quality, nutritional and organoleptic improvements of dairy analogues produced with the present LAB.

### 2.2 DNA fingerprinting using RAPD analysis

A RAPD analysis was performed using DNA from *L. casei* T (LMG P-23506) adapted to a S medium and from *L. casei* T adapted to a MRS medium. For a definition of the S and MRS media, see 2.1 above. -

Results of this analysis revealed that the bacterial cultures did not show differences in their DNA finger print for the selected primers. Results further indicated that adapting the bacteria to a S medium compared to a MRS medium did not induce any differences on a DNA level (mutations) for the selected primers, but as further shown under point 2.1 above, resulted in differences in enzyme activity and induction patterns and thus in adaptation.

### 2.3 Protein patterns using SDS-page

A one-dimensional SDS-PAGE analysis was performed on *Lactobacillus casei* LMG 6904, and *L*. *casei* T (LMG P-23506) adapted to a S medium and on *L. casei* T adapted to a MRS medium.

The protein pattern of the *L*. *casei* T (LMG P-23506) adapted to the S medium was different from the protein pattern of the other two bacterial cultures, pointing at a difference in enzyme activity between the studied bacterial cultures due to adaptation.

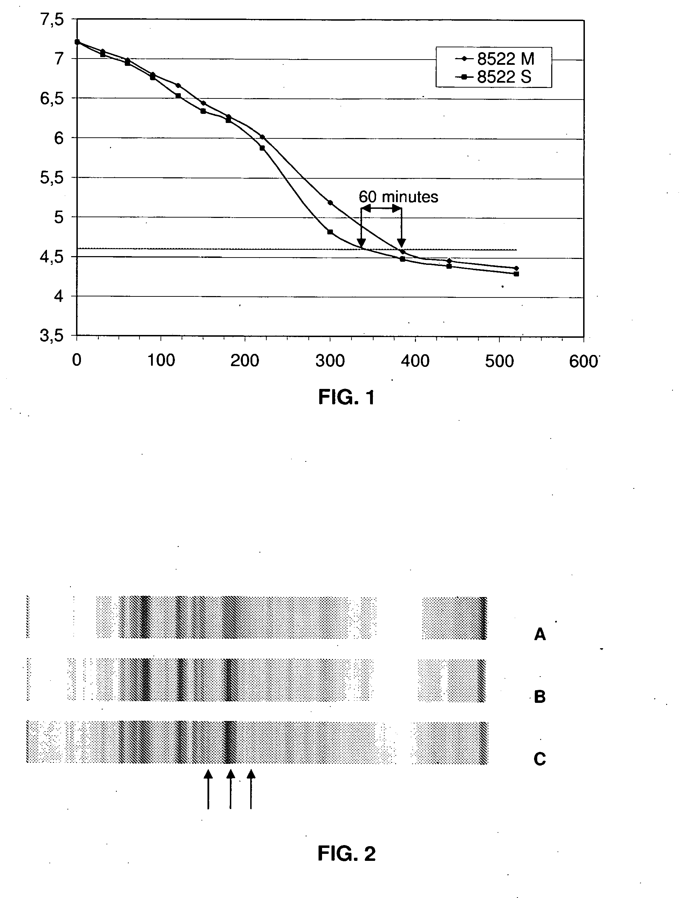

**FIG. 2** illustrates the differences in protein expression. The arrows point to differences in specific protein expressions. As is shown by comparison of **FIG. 2A** showing the SDS PAGE profile of *L*. *casei* T on S medium and **FIG. 2B** showing the SDS PAGE profile of *L*. *casei* T on MRS medium, differences in specific protein expressions occur by adaptation to the respective adaptation medium. As is shown by comparison of **FIG. 2A** showing the SDS PAGE profile of *L*. *casei* T on S medium and **FIG. 2C** showing the SDS PAGE profile of *L*. *casei* 6904, the adapted strain *L*. *casei* T has become different from the parent strain *L*. *casei* 6904 which was obtained from the culture collection BCCM.

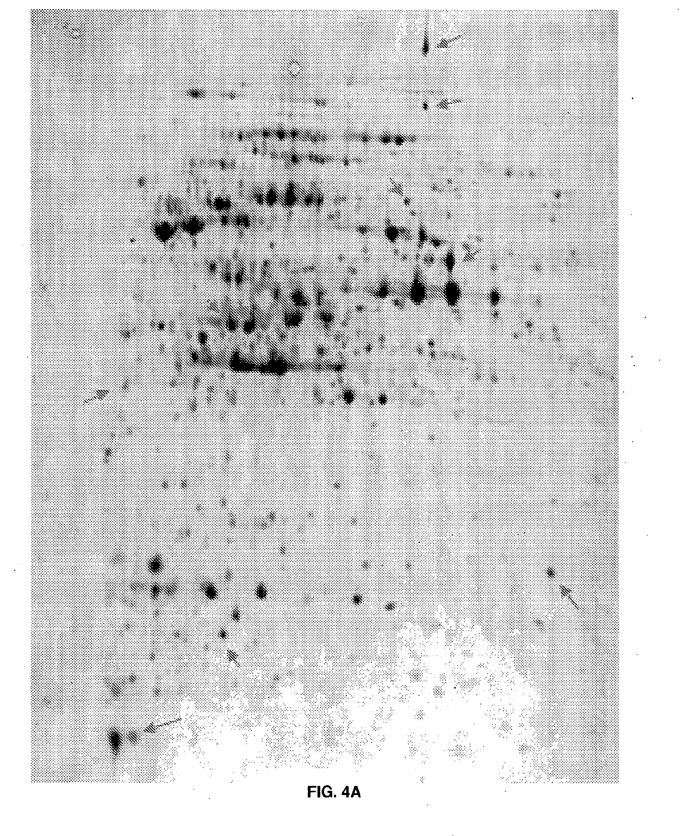

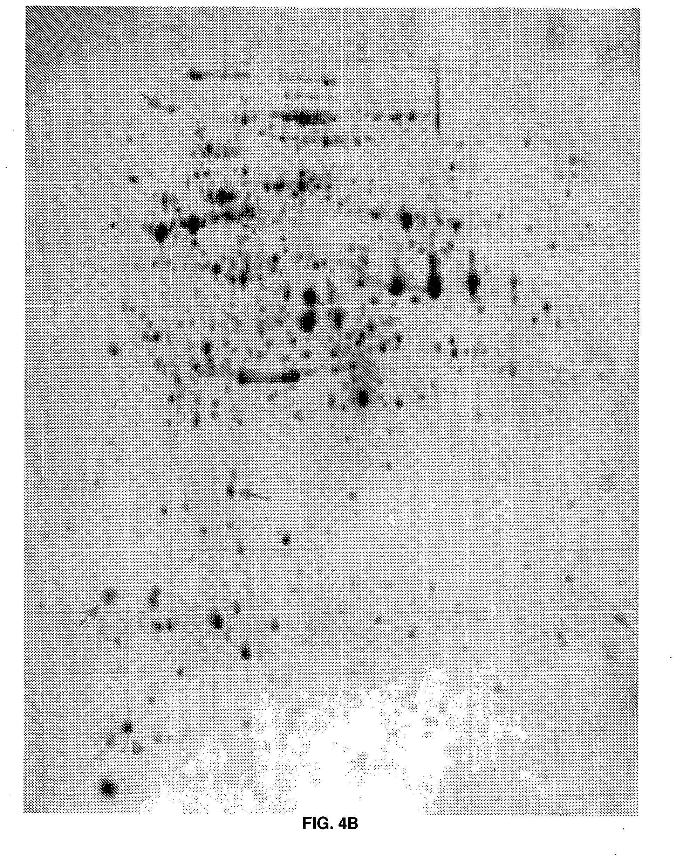

In another example, a two-dimensional SDS page analysis was performed in which proteins were separated according to their size and iso-electric point (iso-electric focussing). **Figure 4** illustrates the results of such analysis. The results of this 2D analysis for *L*. *casei* V (LMG-23504) grown on S medium (**FIG. 4A**) or MRS medium (**FIG. 4B**) demonstrate the difference in protein expression between the two samples. On **FIG. 4** the arrows indicate the differences between both gels.

### 2.4 Total cell content analysis (FTIR-UATR) (Perkin Elmer)

Using Infra Red Spectroscopy, the total cell content of four bacterial cultures was studied. The cell content is considered as a characteristic for a certain strain under certain growth conditions. Using databases, it is possible to identify bacteria using IR spectra.

In this experiment *Lactobacillus casei* V (LMG P-23504), *L*. *casei T* (LMG P-23506), *Lactococcus lactis* S (LMG P-23669) and *Lactococcus lactis* LMG 9452 were grown and maintained in several ways including: 1) adapted to S medium; 2) adapted to MRS medium; 3) adapted to S medium and re-adapted to MRS medium; and 4) adapted to MRS medium and re-adapted to S medium.

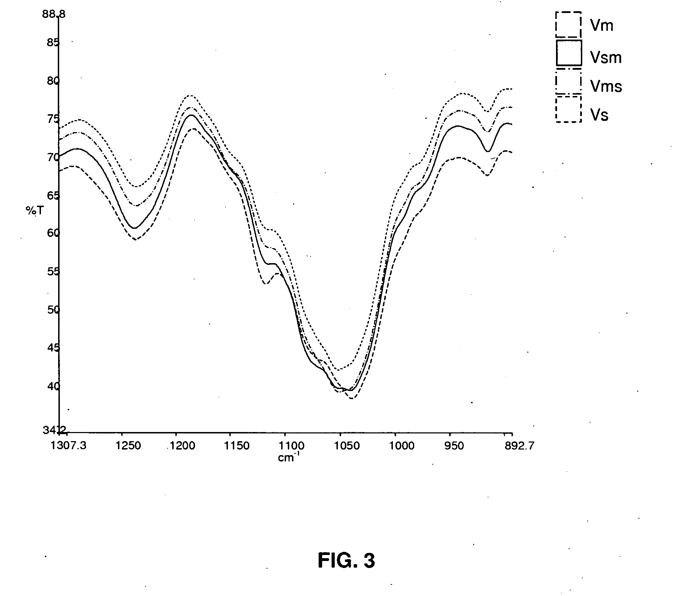

Results of this experiment showed significant differences between bacteria that were adapted to S medium and bacteria that were adapted to MRS medium. Results are expressed in % similarity (with a standard deviation of ≤1 %). The difference between *Lactobacillus casei* V (LMG-23504) adapted to S medium and adapted to MRS was 3.5 %. When the L. casei V adapted to S (VS) was re-adapted to MRS (VSM) the difference was 1.4 %. When the *L*. *casei* V adapted to MRS (VM) was re-adapted to S medium (VMS), the difference was 3%. **FIG. 3** shows a gradual transition in typical IR spectrum when comparing VS, VSM, VMS and VM.

The difference between *L*. *casei T* (LMG P-23506) adapted to S medium and adapted to MRS was 3.3 %.

The difference between *Lactococcus lactis* S (LMG P-23669) adapted *to* S medium and adapted to MRS was 4.3 %.

The difference between *Lactococcus lactis* LMG 9452 adapted to S medium and adapted to MRS was 3.1 %.

### Example 3: Fermentation studies

The following examples illustrate that the use of a culture of lactic acid bacteria that have been isolated, adapted and cultured according to the present invention permits to improve the preparation of dairy analogue, and in particular to improve (accelerate) its fermentation and thus to reduce fermentation time during the preparation of a dairy analogue.

### * Lactococcus lactis S (LMG P-23669)

Bacteria cultures of *Lactococcus lactis* S (LMG P-23669) were prepared according to the present invention by culturing said strain on an S medium or on an MRS medium, as defined in example 2. Similar inoculation amounts, comparable to McFarland scale n° 5, of these bacteria cultures were then used for the fermentation of a soy based composition consisting of 85 % by weight of soy milk, 2% by weight of fructose and 13% by weight of water. The time necessary to obtain a pH value of 4.6 was determined. Such pH value is considered as a "safe" value for suppressing the development of pathogens. As illustrated on **FIG. 1****,** the time required for reaching this pH value using a bacteria culture of *Lactococcus lactis* S cultured to S medium was approximately 60 minutes shorter than when using a bacteria culture of *Lactococcus lactis* S cultured on an MRS medium. Results of this experiment thus indicated that the use of LAB that were grown on a substantially vegetable medium according to the invention permits to reduce times required for the preparation (fermentation) of dairy analogues. In this example the time required for reaching a pH value which is considered as "safe" for suppressing the development of pathogens is reached about 1.2 times faster when using a *Lactococcus* strain that has been isolated, adapted and cultured on a 100% vegetable medium. This example demonstrates the technological advantage of adaptation of the bacteria strain to the S medium. Fermentation time during the preparation of said dairy analogue can be reduced with about 20%.

### * Lactococcus lactis S (LMG P-23669)

Bacteria cultures of *Lactococcus lactis* S (LMG P-23669) were prepared according to the present invention by culturing said strain on an S medium or on an MRS medium, as defined in example 2.

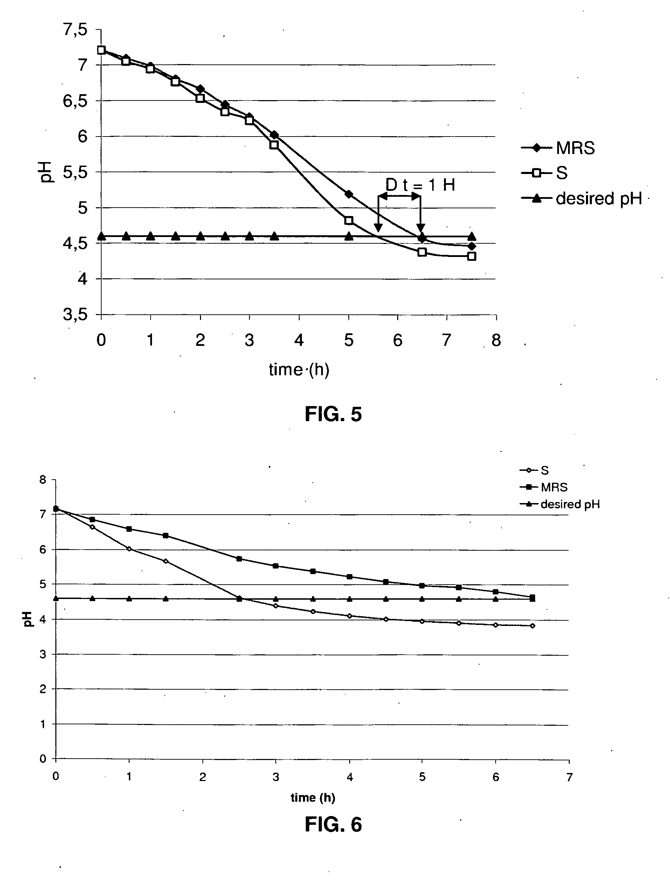

Soy milk with 2% fructose was inoculated with 1X10⁷/ mL of *Lactococcus lactis* adapted to S or MRS medium and fermented at 30° until the critical pH of 4.6 was reached. **Figure 5** shows that *L. lactis* S adapted to S medium reached the pH of 4.6 one hour earlier than the traditionally grown MRS bacteria. In this example the time required for reaching a pH value which is considered as "safe" for suppressing the development of pathogens is reached about 1.2 times faster when using a *Lactococcus* strain that has been isolated, adapted and cultured on a 100% vegetable medium. Fermentation time during the preparation of said dairy analogue can be reduced with about 20%. This example demonstrates the technological advantage of adaptation of the bacteria strain to the S medium.

### * Lactobacillus casei V (LMG P-23504)

Bacteria cultures of *Lactobacillus casei* V (LMG P-23504) were prepared according to the present invention by culturing said strain on an S medium or on an MRS medium, as defined in example 2.

Soy milk with 2% added glucose was inoculated with 5X10⁷/ mL *L*. *casei* V grown on S or MRS medium. The fermentation was performed at 37° until the safe pH limit of 4.6 was reached. **Figure 6** shows that the *L*. *casei* V that was adapted to the S medium already reached the desired pH of 4.6 after 2.5 hours of fermentation, while the MRS grown *L*. *casei* V reached this pH after 6.5 hours of fermentation. The time required for reaching this pH value using a bacteria culture of *Lactobacillus casei* V cultured on S medium was approximately 4 hours shorter than when using a bacteria culture of *Lactobacillus casei* V cultured on an MRS medium. Thus, the time required for reaching a pH value which is considered as "safe" for suppressing the development of pathogens is reached more than 2 times faster, and in this example 2.6 times faster when using a *Lactobacillus* strain that has been isolated, adapted and cultured on a 100% vegetable medium. Fermentation time during the preparation of said dairy analogue can be reduced with more than 100%. This example demonstrates the technological advantage of adaptation of the bacteria strain to the S medium.

### * Bifidobacterium infantis (LMG P-24096)

Bacteria cultures of *Bifidobacterium infantis* (LMG P-24096) were prepared according to the present invention by culturing said strain on an S medium or on an MRS medium, as defined in example 2.

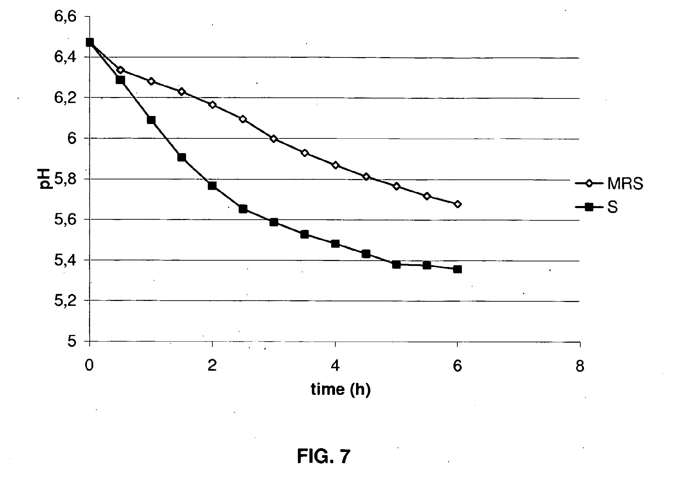

Soy milk with 2.5 % raffinose was inoculated with 1X10⁷/ mL of *B. infantis* adapted to S or MRS medium and fermented at 37° under anaerobic conditions. **Figure 7** shows that *B*. *infantis* adapted to the S medium grows faster than the MRS culture. After 6 hours the fermentation was stopped because the *B. infantis* inhibits itself due to a decreasing pH.

During fermentation *B. infantis* inhibits itself due to a decreasing pH and the formation of lactic and acetic acid. To examine the acid forming capacity without an inhiting effect of the decreasing pH, the fermentation test described above was repeated but the pH was kept constant at 5.9 by addition of NaOH 1 M.

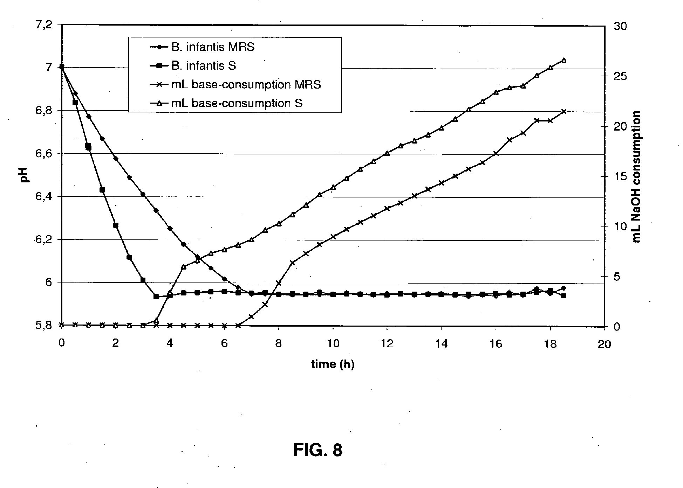

**Figure 8** demonstrates that the *B. infantis* that was adapted to S medium reached a pH of 5.9 approximately 3.5 hours earlier than *B. infantis* that was adapted to MRS medium and that more NaOH was consumed during fermentation. This demonstrates the higher speed of growth and the higher acid forming capacity of *B. infantis,* when adapted to S medium. Thus, the time required for reaching a pH value of 5.9 is reached more than 2 times faster, and in this example 2.2 times faster when using a *B. infantis* strain that has been isolated, adapted and cultured on a 100% vegetable medium. This example demonstrates the technological advantage of adaptation of the bacteria strain to the S medium.

## Claims

1. A method for preparing a dairy analogue comprising the steps of:
a) isolating one or more lactic acid bacteria from their natural environment or from a culture collection on a suitable animal-free isolation medium;
b) adapting said isolated bacteria by growing said bacteria on a suitable animal-free adaptation medium;
c) culturing said adapted bacteria in a suitable animal-free culture medium, and
d) preparing a dairy analogue by adding to said dairy analogue as a starting material a suitable amount of bacteria obtained in step c);
wherein steps a) to d) are carried out under animal-free conditions.

2. Method according to claim 1, wherein step d) comprises fermenting a dairy analogue by adding to said dairy analogue as a starting material a suitable amount of bacteria obtained in step c).

3. Method according to claim 1 or 2, wherein said animal-free isolation, adaptation and culture medium are 100% vegetable media.

4. Method according to any of claims 1 to 3, wherein said isolation, adaptation and culture media comprise between 0.1 and 10 % by weight of a vegetable peptone, preferably a papaic digest of soybean protein.

5. Method according to any of claims 1 to 4, wherein said media further comprise between 0.1 and 10 % by weight of a yeast component, preferably an extract of yeast cells.

6. Method according to any of claims 1 to 5, wherein said media further comprise between 0.1 and 10 % by weight of a buffering agent, preferably a phosphate buffer, that maintains said medium at a pH of between 5 and 8.

7. Method according to any of claims 1 to 6, wherein said media further comprise between 0.1 and 10% by weight of a vegetable derived fermentable carbohydrate.

8. Method according to any of claims 1 to 7, wherein said lactic acid bacteria are selected from the genera *Lactobacillus*, *Lactococcus, Bifidobacterium* and *Streptococcus.*

9. Method according to claim 8, wherein said lactic acid bacteria are selected from the group comprising *Lactobacillus casei*, *Lactobacillus paracasei,* or *Lactococcus lactis,* or *Streptococcus salivarius* spp. *thermophilus.*

10. Method according to claim 8 or 9, wherein said lactic acid bacteria comprise *Lactobacillus casei* V (LMG P-23504), *Lactobacillus casei* W (LMG P-23505), *Lactobacillus casei* T (LMG P-23506), *Lactococcus lactis* S (LMG P-23669) *Bifidobacterium infantis* S (LMG P-24096) and/or *Streptococcus salivarius* ssp. *thermophilus* (LMG P-24095).

11. Method according to any of claims 1 to 10, wherein said dairy analogue is a vegetable food product, a vegetable food ingredient, or a vegetable functional food.

12. Method according to claim 11, wherein said dairy analogue is derived from soya.

13. Method according to any of claims 2 to 12, wherein fermentation time during the preparation of said dairy analogue is reduced.

14. A dairy analogue obtainable by the method of any of claims 1 to 13.

15. Dairy analogue according to claim 14, having a reduced amount of phytic acid.

16. Dairy analogue according to claim 14 or 15, having a reduced amount of raffinose.

17. Dairy analogue according to any of claims 14 to 16, having an increased amount of aglucon forms of isoflavones.

18. A bacteria culture comprising one or more lactic acid bacteria that have been isolated and adapted by growing on a 100% vegetable isolation and adaptation medium, respectively.

19. Lactic acid bacteria that have been isolated and adapted by growing on a 100% vegetable isolation and adaptation medium, respectively.

20. Lactic acid bacteria according to claim 19, showing an increase in activity of one or more enzymes compared to lactic acid bacteria that have not been grown on a 100% vegetable isolation and adaptation medium.

21. Lactic acid bacteria according to claim 20, wherein said enzyme(s) is (are) selected from the group comprising acid phosphatase, alkaline phosphatase, α-chymotrypsin, α-galactosidase and β-glucosidase.

22. A bacteria culture according to claim 18 or lactic acid bacteria according to any of claims 19-21, wherein said vegetable medium is as defined as in any of claims 3-7.

23. A bacteria culture according to claim 18 or lactic acid bacteria according to any of claims 19-21 wherein said lactic acid bacteria are as defined as in any of claims 8-10.

24. Use of a bacteria culture according to any of claims 18, 22 or 23 or lactic acid bacteria according to any of claims 19-21 for improving the preparation of dairy analogue.

25. Use of a bacteria culture according to any of claims 18, 22 or 23 or lactic acid bacteria according to any of claims 19-21 for reducing fermentation time during the preparation of a dairy analogue.

## Patentansprüche

1. Verfahren zur Herstellung eines Milchproduktanalogs, umfassend die folgenden Schritte:
a) Isolieren einer oder mehrerer Milchsäurebakterien aus ihrer natürlichen Umgebung oder aus einer Kultursammlung auf einem geeigneten tierfreien Isolierungsmedium;
b) Adaptieren der isolierten Bakterien durch Züchten der Bakterien auf einem geeigneten tierfreien Adaptionsmedium;
c) Züchten der adaptierten Bakterien in einem geeigneten tierfreien Kulturmedium, und
d) Herstellen eines Milchproduktanalogs durch Zugabe einer geeigneten Menge der Bakterien, die in Schritt c) erhalten wurden, zu dem Milchproduktanalog als Ausgangsmaterial;
wobei die Schritte a) bis d) unter tierfreien Bedingungen ausgeführt werden.

2. Verfahren nach Anspruch 1, wobei Schritt d) das Fermentieren eines Milchproduktanalogs durch Zugabe einer geeigneten Menge der Bakterien, die in Schritt c) erhalten wurden, zu dem Milchproduktanalog als Ausgangsmaterial umfasst.

3. Verfahren nach Anspruch 1 oder 2, wobei das tierfreie Isolierungs-, Adaptions- und Kulturmedium 100% pflanzliche Medien sind.

4. Verfahren nach einem der Ansprüche 1 bis 3, wobei die Isolierungs-, Adaptions- und Kulturmedien 0,1 bis 10 Gewichtsprozent eines pflanzlichen Peptons, vorzugsweise eines Papainhydrolysats von Sojabohnenprotein enthalten.

5. Verfahren nach einem der Ansprüche 1 bis 4, wobei die Medien des Weiteren 0,1 bis 10 Gewichtsprozent einer Hefekomponente, vorzugsweise eines Extrakts von Hefezellen umfassen.

6. Verfahren nach einem der Ansprüche 1 bis 5, wobei die Medien des Weiteren 0,1 bis 10 Gewichtsprozent eines Puffermittels, vorzugsweise eines Phosphatpuffers, umfassen, das das Medium bei einem pH-Wert zwischen 5 und 8 hält.

7. Verfahren nach einem der Ansprüche 1 bis 6, wobei die Medien des Weiteren 0,1 bis 10 Gewichtsprozent eines von Pflanzen abgeleiteten fermentierbaren Kohlenhydrates umfassen.

8. Verfahren nach einem der Ansprüche 1 bis 7, wobei die Milchsäurebakterien ausgewählt sind aus den Gattungen *Lactobacillus, Lactococcus, Bifidobacterium* und *Streptococcus*.

9. Verfahren nach Anspruch 8, wobei die Milchsäurebakterien ausgewählt sind aus der Gruppe, die *Lactobacillus casei, Lactobacillus paracasei* oder *Lactobacillus lactis* oder *Streptococcus salivarius spp*. *thermophilus* umfasst.

10. Verfahren nach Anspruch 8 oder 9, wobei die Milchsäurebakterien *Lactobacillus casei* V (LMG P-23504), *Lactobacillus casei* W (LMG P-23505), *Lactobacillus casei* T (LMG P-23506), *Lactococcus lactis* S (LMG P-23669), *Bifidobacterium infantis* S (LMG P-24096) und/oder *Streptococcus salivarius spp*. *thermophilus* (LMG P-24095) umfassen.

11. Verfahren nach einem der Ansprüche 1 bis 10, wobei das Milchproduktanalog ein pflanzliches Nahrungsmittelprodukt, ein pflanzlicher Nahrungsmittelinhaltsstoff oder ein pflanzliches funktionelles Nahrungsmittel ist.

12. Verfahren nach Anspruch 11, wobei das Milchproduktanalog von Soja abgeleitet ist.

13. Verfahren nach einem der Ansprüche 2 bis 12, wobei die Fermentationszeit während der Herstellung des Milchproduktanalogs verkürzt ist.

14. Milchproduktanalog, das durch das Verfahren nach einem der Ansprüche 1 bis 13 erhältlich ist.

15. Milchproduktanalog nach Anspruch 14 mit einer verringerten Menge an Phytinsäure.

16. Milchproduktanalog nach Anspruch 14 oder 15 mit einer verringerten Menge an Raffinose.

17. Milchproduktanalog nach einem der Ansprüche 14 bis 16 mit einer erhöhten Menge an Agluconformen von Isoflavonen.

18. Bakterienkultur, umfassend eine oder mehr Milchsäurebakterien, die durch Züchten auf einem 100% pflanzlichen Isolierungs- und Adaptionsmedium isoliert beziehungsweise adaptiert wurden.

19. Milchsäurebakterien, die durch Züchten auf einem 100% pflanzlichen Isolierungs- und Adaptionsmedium isoliert beziehungsweise adaptiert wurden.

20. Milchsäurebakterien nach Anspruch 19, die eine Zunahme in der Aktivität eines Enzyms oder mehrerer Enzyme im Vergleich zu Milchsäurebakterien zeigen, die nicht auf einem 100% pflanzlichen Isolierungs- und Adaptionsmedium gezüchtet wurden.

21. Milchsäurebakterien nach Anspruch 20, wobei das Enzym oder die Enzyme ausgewählt ist beziehungsweise sind aus der Gruppe umfassend Säurephosphatase, alkalische Phosphatase, α-Chymotrypsin, α-Galactosidase und β-Glucosidase.

22. Bakterienkultur nach Anspruch 18 oder Milchsäurebakterien nach einem der Ansprüche 19 bis 21, wobei das pflanzliche Medium wie in einem der Ansprüche 3 bis 7 definiert ist.

23. Bakterienkultur nach Anspruch 18 oder Milchsäurebakterien nach einem der Ansprüche 19 bis 21, wobei die Milchsäurebakterien wie in einem der Ansprüche 8 bis 10 definiert sind.

24. Verwendung einer Bakterienkultur nach einem der Ansprüche 18, 22 oder 23 oder von Milchsäurebakterien nach einem der Ansprüche 19 bis 21 zur Verbesserung der Herstellung eines Milchproduktanalogs.

25. Verwendung einer Bakterienkultur nach einem der Ansprüche 18, 22 oder 23 oder von Milchsäurebakterien nach einem der Ansprüche 19 bis 21 zur Verkürzung der Fermentationszeit in der Herstellung eines Milchproduktanalogs.

## Revendications

1. Procédé de préparation d'un analogue de produit laitier, qui comprend les étapes consistant à :
a) isoler une ou plusieurs bactéries de l'acide lactique de leur environnement naturel ou d'une collection de culture sur un milieu d'isolation adapté et dépourvu de composant d'origine animale ;
b) adapter lesdites bactéries isolées en cultivant lesdites bactéries sur un milieu d'adaptation adapté, dépourvu de composant d'origine animale ;
c) cultiver lesdites bactéries adaptées dans un milieu de culture adapté, dépourvu de composant d'origine animale, et
d) préparer un analogue de produit laitier par ajout au dit analogue de produit laitier sous la forme d'un matériau de départ d'une quantité adaptée de bactéries obtenues dans l'étape c) ;
dans lequel les étapes a) à d) sont réalisées dans des conditions d'absence de composant d'origine animale.

2. Procédé selon la revendication 1, dans lequel l'étape d) comprend la fermentation d'un analogue de produit laitier par ajout au dit analogue de produit laitier sous la forme d'un matériau de départ d'une quantité adaptée de bactéries obtenues dans l'étape c).

3. Procédé selon la revendication 1 ou 2, dans lequel lesdits milieux d'isolation, d'adaptation et de culture dépourvus de composant d'origine animale sont des milieux 100 % végétaux.

4. Procédé selon l'une quelconque des revendications 1 à 3,
dans lequel lesdits milieux d'isolation, d'adaptation et de culture comprennent de 0,1 à 10 % en poids d'une peptone végétale, de préférence un produit de digestion papaïque de la protéine du soja.

5. Procédé selon l'une quelconque des revendications 1 à 4,
dans lequel lesdits milieux comprennent en outre de 0,1 à 10 % en poids d'un composant de levure, de préférence d'un extrait de cellules de levure.

6. Procédé selon l'une quelconque des revendications 1 à 5,
dans lequel lesdits milieux comprennent en outre de 0,1 à 10 % en poids d'un agent tampon, de préférence d'un tampon phosphate, qui maintient lesdits milieux à un pH compris entre 5 et 8.

7. Procédé selon l'une quelconque des revendications 1 à 6,
dans lequel lesdits milieux comprennent en outre de 0,1 à 10 % en poids d'un glucide d'origine végétale pouvant fermenter.

8. Procédé selon l'une quelconque des revendications 1 à 7,
dans lequel lesdites bactéries de l'acide lactique sont choisies dans parmi les genres *Lactobacillus, Lactococcus, Bifidobacterium* et *streptococcus.*

9. Procédé selon la revendication 8, dans lequel lesdites bactéries de l'acide lactique sont choisies dans le groupe comprenant *Lactobacillus casei, Lactobacillus paracasei,* ou *Lactococcus lactis,* ou *Streptococcus salivarius* ssp. *thermophilus.*

10. Procédé selon la revendication 8 ou 9, dans lequel lesdites bactéries de l'acide lactique comprennent Lactobacillus casei V (LMG P-23504), *Lactobacillus casei* W (LMG P-23505), *Lactobacillus casei* T (LMG P-23506), *Lactococcus lactis* S (LMG P-23669) *Bifidobacterium infantis* S (LMG P-24096) et/ou *Streptococcus salivarius* ssp. *thermophilus* (LMG P-24095).

11. Procédé selon l'une quelconque des revendications 1 à 10,
dans lequel ledit analogue de produit laitier est un produit alimentaire végétal, un ingrédient alimentaire végétal, ou un aliment fonctionnel végétal.

12. Procédé selon la revendication 11, dans lequel ledit analogue de produit laitier est dérivé du soja.

13. Procédé selon l'une quelconque des revendications 2 à 12,
dans lequel la durée de fermentation pendant la préparation dudit analogue de produit laitier est réduite.

14. Analogue de produit laitier pouvant être obtenu par le procédé selon l'une quelconque des revendications 1 à 13.

15. Analogue de produit laitier selon la revendication 14, ayant une quantité réduite d'acide phytique.

16. Analogue de produit laitier selon la revendication 14 ou 15, ayant une quantité réduite de raffinose.

17. Analogue de produit laitier selon l'une quelconque des revendications 14 à 16, ayant une quantité accrue de formes aglucones d'isoflavones.

18. Culture bactérienne comprenant une ou plusieurs bactéries de l'acide lactique qui ont été isolées et adaptées par culture sur un milieu d'isolation et d'adaptation 100 % végétal, respectivement.

19. Bactéries de l'acide lactique qui ont été isolées et adaptées par culture sur un milieu d'isolation et d'adaptation 100 % végétal, respectivement.

20. Bactéries de l'acide lactique selon la revendication 19, présentant une augmentation de l'activité d'une ou plusieurs enzymes comparées à des bactéries de l'acide lactique qui n'ont pas été cultivées sur un milieu d'isolation et d'adaptation 100 % végétal.

21. Bactéries de l'acide lactique selon la revendication 20,
dans lesquelles ladite (lesdites) enzyme(s) est (sont) choisie(s) dans le groupe comprenant la phosphatase acide, la phosphatase alcaline, l'α-chymotrypsine, l'a-galactosidase et la β-glucosidase.

22. Culture bactérienne selon la revendication 18, ou bactéries de l'acide lactique selon l'une quelconque des revendications 19 à 21, dans laquelle ledit milieu végétal est tel que défini dans l'une quelconque des revendications 3 à 7.

23. Culture bactérienne selon la revendication 18, ou bactéries de l'acide lactique selon l'une quelconque des revendications 19 à 21, dans laquelle lesdites bactéries de l'acide lactique sont telles que définies dans l'une quelconque des revendications 8 à 10.

24. Utilisation d'une culture bactérienne selon l'une quelconque des revendications 18, 22 ou 23, ou de bactéries de l'acide lactique selon l'une quelconque des revendications 19 à 21, en vue d'améliorer la préparation d'un analogue de produit laitier.

25. Utilisation d'une culture bactérienne selon l'une quelconque des revendications 18, 22 ou 23, ou de bactéries de l'acide lactique selon l'une quelconque des revendications 19 à 21, en vue de réduire la durée de fermentation pendant la préparation d'un analogue de produit laitier.
